# EUROPEAN PATENT APPLICATION

(11) **EP 2 476 487 A1**
(43) Date of publication of application: **18.07.2012**
(21) Application number: 10762705.1
(22) Date of filing: 12.08.2010
(51) Int. Cl.: B01J 23/887, B01J 37/10, B01J 37/03, B01J 37/02, B01J 37/00, B01J 37/08, B01J 37/20, C07C 1/04, C07C 27/06, C07C 29/15, C07C 31/08

(54) **METHOD FOR OBTAINING A MULTIMETALLIC SULFUREOUS CATALYST AND USE THEREOF IN A METHOD FOR PRODUCING HIGHER ALCOHOLS BY CATALYTIC CONVERSION OF SYNTHESIS GAS**

(30) Priority: 10.09.2009 ES 200930675
(71) Applicant: Abengoa Bioenergía Nuevas Tecnologías, S. A., 41018 Sevilla (ES)
(72) Inventor: PRIETO GONZÁLEZ, Gonzalo, E-41018 Sevilla (ES); SERRA ALFARO, José Manuel, E-41018 Sevilla (ES); MARTÍNEZ FELIU, Agustín, E-41018 Sevilla (ES); SANZ YAGÜE, Juan Luis, E-41018 Sevilla (ES); CARABALLO BELLO, José, E-41018 Sevilla (ES); ARJONA ANTOLÍN, Ricardo, E-41018 Sevilla (ES)
(74) Representative: Pons Ariño, Angel
(86) International application number: PCT/ES2010/070553
(87) International publication number: WO 2011/029973

(57) **Abstract**

The present invention relates to a sulphided multi-metallic catalyst, the process for obtaining it by sulphidation of a multi-metallic solid and use thereof in a process for producing higher alcohols (C₂₊), mainly ethanol, through the catalytic conversion of synthesis gas.

## Description

The present invention relates to a process for obtaining a sulphided multi-metallic catalyst and use thereof in a process for producing higher alcohols (C₂₊), mainly ethanol, through the catalytic conversion of synthesis gas. Additionally, the invention relates to the catalyst obtained by means of said process and to its use in obtaining alcohols.

### STATE OF THE ART

At present, the use of higher alcohols (C₂₊), mainly ethanol, as petrol additives or directly as fuels for internal combustion engines or fuel cells has increased significantly. Ethanol is considered the best candidate for counteracting the exhaustion of petroleum reserves, as a main fuel source, and the stringent environmental policies relative to transport fuels.

The production of ethanol and other higher alcohols through the catalytic conversion of synthesis gas (CO+H₂) allows the valorisation of abundant material sources such as natural gas reserves or renewable sources such as different types of biomass.

The activity of heterogeneous catalysts based on molybdenum sulphide (MoS₂), promoted by alkaline functions and optionally co-promoted by transition metals, in the conversion of synthesis gas in higher alcohols, is known.

Patents WO8503073 and US5102845 disclose the use of catalysts consisting mainly of molybdenum sulphide (Mo) promoted by one or several alkaline metals and, optionally, co-promoted by tantalum (Ta). Catalysts are obtained through a process that initially comprises the thermal decomposition of a sulphided molybdenum precursor (ammonium thiomolybdate (NH₄)₂MoS₄) at temperatures of between 300ºC and 600ºC, giving rise to molybdenum sulphide (MoS₂); subsequently, the promoter elements consisting of a group 1 element of the periodic table, and optionally Ta, are preferably introduced by impregnation of MoS₂ with aqueous solutions of promoter salts and, finally, the catalyst is thermally activated in the absence of sulphur. These catalysts are applied to the catalytic conversion of synthesis gas at temperatures of between 300ºC and 350ºC and a pressure of 2.8 MPa, obtaining selectivities to ethanol of 30-47% at CO conversion levels of 0.5-4%.

Patents US4675344, US4749724, US4752623, US4882360 and US4831060 disclose the use of catalysts consisting mainly of molybdenum sulphide (Mo) or tungsten (W), promoted by one or several alkaline groups (group 1) or alkaline earth metals (group 2) and optionally co-promoted by transition metals (groups 8, 9 and 10). Supported catalysts are obtained by impregnation of the catalytic supports with metal precursor solutions and subsequent thermal activation and sulphidation. In the case of massive catalysts (not supported) in a particular embodiment of the disclosed processes, these are obtained through a process that initially comprises the thermal decomposition of a sulphided molybdenum precursor (ammonium thiomolybdate (NH₄)₂MoS₄) at temperatures of between 300ºC and 600ºC, giving rise to molybdenum sulphide (MoS₂). In particular embodiments of the disclosed processes, the catalyst is prepared by coprecipitation of a multi-metallic solid by adding aqueous solutions of a sulphided molybdenum precursor (ammonium thiomolybdate) and soluble precursors of metal promoters (generally acetates) to an acidified solution of acetic acid and the precipitate obtained is calcined in nitrogen at 500ºC. Massive catalysts obtained by any of the embodiments mentioned in the patents have a sulphided nature at this point of the preparation process. An alkaline or alkaline earth metal promoter is added to the sulphided catalysts by means of aqueous impregnation or solid-state physical mixture processes and thermally activated, in the absence of sulphur. Catalysts thus prepared are applied to the catalytic conversion of synthesis gas at temperatures of between 300ºC and 320ºC and a pressure of 10.45 MPa, using synthesis gas with a mole ratio of H₂/CO = 0.98-1.06 and giving rise to selectivities to ethanol of 25-39% (CO₂-free carbon base) at CO conversion levels of 30-39%.

More recently, patent US6248796 has disclosed a process for synthesizing a catalyst based on molybdenum sulphide (Mo), tungsten (W) or chrome (Cr). The process for obtaining the catalyst comprises an ultrasound treatment of a solution containing a metal carbonyl as a precursor, giving rise to a nanometric crystal size for the solid obtained. Sulphidation of the catalyst, using a compound as a sulphur source, may take place during synthesis thereof in the presence of ultrasound or subsequent thereto. The catalyst is applied to the synthesis of alcohols from synthesis gas.

On the other hand, it has been disclosed that the nature of the active centres and the catalytic activity of molybdenum sulphide catalysts (Mo) and/or tungsten (W), applied in desulphidation and/or denitrification reactions of hydrocarbon streams in the presence of hydrogen, is very sensitive to the nature (crystalline phase, composition, texture) of the non-sulphided solids that will subsequently give rise to sulphided catalysts after sulphidation treatment (P. Arnoldy et al., J. Catal. 92 (1985) 35). Patents US6299760, US6635599, US7232515 and patent application WO2007/048593 A1 disclose the preparation of sulphided catalysts containing at least one main metal belonging to group 6 of the periodic table (Cr, Mo, W) promoted by transition metals from groups 7, 8, 9 and 10. The process for obtaining the catalyst comprises the precipitation of a solid from the aqueous solutions of the metal components, in an alkaline medium, at temperatures of around 90ºC. Under certain conditions, the solid obtained has a nickel and ammonium molybdate-type crystalline structure ((NH₄)Ni₂(OH)₂(MoO₄)₂), according to the structure disclosed by D. Levin, S.L. Soled, J.Y. Ying, Inorganic Chemistry, Vol. 35 (1996) 4191. The non-sulphided precursor obtained is subsequently activated by thermal treatments that include sulphidation, using a sulphided compound as the sulphur source. According to the aforementioned documents, massive catalysts thus prepared are applied to hydrotreatment reactions of hydrocarbon streams, such as hydrodesulphidation and/or hydrodenitrification.

### DETAILED DESCRIPTION OF THE INVENTION

The present invention relates to a process for obtaining a sulphided multi-metallic catalyst, the catalyst obtained through said process and its use in a process for producing higher alcohols (C₂₊), mainly ethanol, through the catalytic conversion of synthesis gas.

The first aspect of the present invention is a process for obtaining a sulphided multi-metallic catalyst, characterised in that a solid undergoes a sulphidation process, wherein said solid comprises at least the following components:

C(i)C(ii)ₓC(iii)y

being,
C(i) a component (i) selected from the list comprising molybdenum (Mo), tungsten (W) and any combination thereof,
C(ii) a component (ii) selected from the list of elements comprising at least one element of groups 7 to 14 of the periodic table and any combination thereof. Groups 7 to 14 include the following elements: Mn, Tc, Re, Fe, Ru, Os, Co, Rh, lr, Ni, Pd, Pt, Cu, Ag, Au, Zn, Cd, Hg, B, Al, Ga, In, Tl, Si, Ge, Sn and Pb,
C(iii) a component (iii) selected from the list of elements comprising at least groups 1 and 2 of the periodic table, lanthanides and any combination thereof. C(iii) can comprise the following elements: Li, Na, K, Rb, Mg, Ca, Sr, Ba, Y, La, Ce, Pr, Nd, Sm, Eu, Gd, Tb, Dy, Ho, Er, Tm, Tb and Lu,
"x" and "y" the mole ratios of C(ii) and C(iii) with respect to C(i), respectively, "x" being comprised between 0.1-10 and "y" between 0.2-10.

In a particular embodiment of the first aspect of the invention, C(i) comprises at least Mo, while C(ii) comprises Co, Ni or any combination thereof.

The properties of the final catalyst have also been observed to improve if, during synthesis thereof, C(ii) also comprises at least one element selected from the list comprising Re, Ru, Rh, Ir, Zn, Ga, In, Ge, Sn, La, Sm and any combination thereof, preferably Re, Ru, Zn, Ga, La, Sm or any combination thereof.

Preferably, C(iii) comprises an alkaline element, i.e. Li, Na, K, Rb, Cs or any combination thereof. More preferably, C(iii) comprises K, Cs or any combination thereof.

The sulphidation of the solid that comprises C(i)C(ii)ₓC(iii)_{y} is carried out to fully or partially transform the solid into a multi-metallic sulphur. Sulphidation can be carried out using any of the methods known to a person skilled in the art, but will preferably by carried out through exposure to a gas stream that comprises a sulphurated component.

"Sulphurated component" shall be understood to be a chemical component that comprises sulphur in its molecular formula. An adequate sulphurated compound for carrying out sulphidation is capable of reacting with the solid in such a manner as to contribute sulphur to form a solid sulphide. In a preferred embodiment of the process of the invention, during the sulphidation treatment, the solid is exposed to a gas stream that comprises a sulphurated compound. This sulphurated compound can be selected from the list that comprises a sulphide with the formula R¹R²S, where R¹ and R² may be identical or different and are selected from among hydrogen, alkyl (C₁-C₆) or aryl; thiophenes such as, by way of non-limiting example, tetrahydrothiophene, methylthiophene, dimethylthiophene, benzothiophene or combinations thereof; mercaptans such as, by way of non-limiting example, methylmercaptan, ethylmercaptan, propylmercaptan, butylmercaptan or combinations thereof; carbonyl sulphide or any combination thereof. More preferably, the sulphurated compound may be hydrogen sulphide (H₂S), a dialkyl sulphide-type compound (R₂S, where R = methyl, ethyl, propyl or benzyl) or a combination thereof and, even more preferably, is H₂S, forming part of a gas stream wherein the molar concentration of the sulphurated compound is comprised between 0.05% and 99%, preferably between 1% and 85% and more preferably between 6% and 20%. The gas stream may also comprise a gas selected from among H₂, synthesis gas, N₂, noble gas (He or Ar) or a combination thereof. This additional gas serves as a carrier of the sulphurated compound.

Sulphidation is carried out preferably at a temperature of between 100ºC and 900ºC, more preferably between 200ºC and 750ºC and, even more preferably, between 300ºC and 600ºC.

In a particular embodiment, the mole ratio "x" is comprised between 0.2 and 2, and more preferably between 0.8 and 1.5.

Preferably, at least 60% of the molar mass of C(ii) is selected from among Co, Ni and any combination thereof. More preferably, at least 80% of the molar mass of C(ii) is selected from among Co, Ni and any combination thereof.

In a particular embodiment, the mole ratio "y" is comprised between 0.1 and 4 and, more preferably, between 0.3 and 1.5.

The solid that comprises C(i)C(ii)ₓC(iii)y obtainable by the process of the present invention, comprises, in a particular embodiment, an element selected from among carbon, nitrogen and combinations thereof. In the event that it contains carbon, the ratio between the moles of carbon and the moles of the (Ci) component will preferably be less than 3 and, more preferably, less than 1.5.

The solid that comprises C(i)C(ii)ₓC(iii)_{y} can be obtained in different manners. In the present invention, the inventors have obtained it by activation, preferably by means of a thermal treatment, of a precursor that comprises: a compound of C(i), a compound of C(ii) and a compound of C(iii). Said compounds may be salts or oxides which are thermally decomposed. Preferably, in said precursor, C(i) and C(ii) form part of the same compound that comprises both C(i) and C(ii). The thermal activation treatment of the precursor is carried out at a temperature comprised between 100ºC and 1,000ºC, preferably between 200ºC and 700ºC and, even more preferably, between 250ºC and 550ºC. In the event that sulphidation is carried out at a subsequent stage, the gas stream used during activation does not contain sulphur or, if it does contain it, the amount is not enough to sulphide the catalyst. The preferred gas streams for carrying out activation are those comprising air, N₂, noble gas, H₂, synthesis gas or any combination thereof.

In a particular embodiment, the solid that comprises C(i)C(ii)ₓC(iii)_{y} obtainable by the process of the present invention is obtained through a process that comprises at least the following stages:
a) Combining and reacting at least one compound of C(i), preferably a salt, with at least one compound of C(ii), preferably a salt, to obtain a solid.
b) Adding a compound of C(iii), preferably a salt, to the solid obtained in stage (a).

Preferably, stage (a) is carried out in a liquid phase, i.e. a liquid is used for the dissolution or dispersion of the compounds of C(i) and C(ii). More preferably, the liquid is an aqueous solution of the compounds of C(i) and C(ii), and during stage (a) a solid precipitates which can be separated by conventional means before adding the compound of C(iii) in stage (b).

In stage (a), the precipitate can occur spontaneously during the combination of the compounds of C(i) and of C(ii) or can be promoted by adjusting the temperature, pH, solvent volume or any combination thereof.

In another particular embodiment, the temperature and pH of an aqueous solution comprising the compound of C(i) are adjusted in order to promote the precipitation of a precipitate when combined with the compound of C(ii). The pH is adjusted preferably between 8 and 13 and, more preferably, between 9 and 11.

In stage (a) of the process of the invention, the pH is adjusted by adding an alkalinizing agent that comprises at least one compound from the list comprising ammonium; ammonium hydroxide, organic amines such as, by way of non-limiting example, methylamine, ethylamine, isopropylamine, propylamine, oleamine, aniline, pyridine and any combination thereof; or compounds which are thermally decomposed, releasing ammonia, such as, by way of non-limiting example, urea, ammonium carbonate and any combination thereof.

When stage (a) takes place in an aqueous medium, the temperature is adjusted to a value comprised between 50ºC and 120ºC, preferably between 70ºC and 100ºC. Stage (a) can be carried out by adding an aqueous solution of the compound of C(ii) to another aqueous solution of the compound of C(i), preferably maintaining the latter solution at a temperature comprised between 50ºC and 120ºC, more preferably between 70ºC and 100ºC.

The compounds of C(i) and C(ii) do not comprise sulphur and can be oxides, complexes with organic ligands or salts. Preferably, said compounds are salts and, more preferably, are soluble in the medium wherein stage (a) takes place. Given that the preferred medium is aqueous, the salts are preferably water soluble. In the case of the compound of C(i), it is preferably ammonium heptamolybdate ((NH₄)₆Mo₇O₂₄), ammonium metatungstate ((NH₄)₆H₂W₁₂O₄₀) or any combination thereof. On the other hand, the compound of C(ii) preferably comprises salts from the list that comprises nitrate, chloride, carbonate, acetate or any combination thereof. The preferred salt of C(ii) is nitrate.

Optionally, a solid support can be suspended in the liquid medium wherein stage (a) of the process of the invention takes place. Support shall be understood to be a solid component which is not indispensable for the catalytic activity but improves certain physical and chemical (such as metallic dispersion) or mechanical characteristics (such as attrition resistance) of the catalyst. This support can comprise an inorganic solid such as, by way of non-limiting example, a metal carbide, an oxide selected from the list that comprises a clay, SiO₂, TiO₂, Al₂O₃, ZrO₂, a lanthanide element oxide or combinations thereof.

It can also comprise carbon in one of its forms such as, by way of non-limiting example, activated carbon, carbon nanofibres, carbon nanotubes or combinations thereof.

Optionally, during stage (a) of the process of the invention a treatment called ageing can be carried out, which consists of a thermal treatment of the precipitated solid suspension, under agitation, at a temperature of between 50ºC and 120ºC, for a time period of between 0.5 and 10 hours, with the objective of ensuring the quantitative precipitate of the solid that comprises C(i) and C(ii).

In a preferred embodiment of the invention, the solid precipitated in stage (a) is isolated by filtration or centrifugation and, optionally, can be dried at a temperature of between 50ºC and 150ºC.

In stage (b) of the process of the invention, which comprises the addition of a compound of C(iii) to the solid obtained in stage (a), said addition can be carried out using a method which may comprise:
- the impregnation of the solid obtained in stage (a) using a solution, preferably aqueous, of one or several compounds of C(iii), preferably one or several salts and, optionally, subsequent drying thereof; or
- the physical mixture in solid state of the precipitate obtained in stage (a) with one or several solid compounds of C(iii), preferably one or several salts.

The salt of C(iii) may be carbonate, hydroxycarbonate, acetate, acetylacetonate, citrate, nitrate, chloride or a combination thereof, and is preferably carbonate, hydroxycarbonate or acetate and, more preferably, carbonate.

A particular, but non-limiting, embodiment of the process of the invention firstly comprises the preparation of a material that comprises C(i) and C(ii), following the synthesis methodology disclosed in M.P. Astier, G. Dji, S.J. Teichner, Ann. Chim. Fr. 12 (1987) 337; D. Levi, S.L. Soled, J.Y. Ying, Inorg. Chem. 35 (1996) 4191; or patent US6299760. Said material is subsequently modified by adding a compound of C(iii), in accordance with stage (b) of the process of the present invention, and is thermally activated in order to obtain the solid that comprises C(i)C(ii)*ₓ*C(iii)*_{y}*, which is sulphided.

In another preferred embodiment of the first aspect of the present invention, the synthesis process of the sulphided multi-metallic catalyst comprises the following stages:
i) preparation of an aqueous solution containing one or several salts of C(i) (solution I);
ii) preparation of a second aqueous solution containing one or several salts of C(ii) (solution II);
iii) adjustment of the pH of solution I to a value comprised between 8 and 13, using an alkalinizing agent;
iv) heating of solution I to a temperature comprised between 50ºC and 120ºC;
v) addition of solution II to solution I, maintaining the latter at a temperature comprised between 50ºC and 120ºC;
vi) ageing, under agitation, of the resulting mixture for a period of time preferably comprised between 0.5 and 5 hours;
vii) isolation of the solid formed from the synthesis waters;
viii) addition of the compound of C(iii) and subsequent drying;
ix) thermal activation of the non-sulphided multi-metallic solid in a gas stream selected from among air, nitrogen or noble gas (He, Ar);
x) sulphidation of the non-sulphided solid, previously thermally activated, in a gas stream flow containing a sulphurated compound selected from among hydrogen sulphide (H₂S), a dialkyl sulphide-type compound (R₂S, where R = methyl, ethyl, propyl, benzyl, etc.), carbonyl sulphide, mercaptans, thiophenes or combinations thereof, in pure form or diluted in a gas which may comprise H₂, synthesis gas, N₂, noble gas (He, Ar) or combinations thereof.

The second aspect of the present invention relates to a catalyst obtainable by the process as defined in the first aspect or in any of its particular embodiments. The catalytic activity of the sulphided catalyst in the conversion of synthesis gas substantially depends on the relative layout of its metal components. The synthesis of a non-sulphided compound which comprises C(i) and C(ii), according to the process of the present invention, gives rise to an intimate mixture of components C(i) and C(ii) resulting, after sulphidationon of the multi-metallic material, in a beneficial technical effect with respect to the catalysts of the state of the art, which are obtained in their sulphided form during the first stages of the synthesis process. This technical effect is a greater selectivity and productivity to higher alcohols (C₂₊) under similar operating conditions.

The catalyst of the present invention can be subjected to milling, mixing with solvents and/or additives for formation thereof, mechanical forming, spray drying-forming, etc. in any of the stages of its preparation. Preferably, the introduction of additives and forming of the catalyst take place before the thermal activation stage.

A third aspect of the present invention relates to the use of the catalyst of the invention in a process for producing higher alcohols (C₂₊) through the catalytic conversion of synthesis gas. Higher alcohols (C₂₊) may comprise ethanol, n-propanol, iso-propanol, n-butanol, iso-butanol, n-pentanol, n-hexanol and combinations thereof. Preferably, the alcohol is ethanol. For the catalytic conversion of synthesis gas, the sulphided multi-metallic catalyst comes into contact with a feed stream that comprises synthesis gas (CO+H₂) and a sulphurated compound, in such a manner that:
- the mole ratio H₂/CO in this stream is comprised between 0.5 and 3, preferably between 0.5 and 2;
- the sulphurated compound may be selected from the list that comprises a sulphide with the formula R¹R²S, wherein R¹ y R² may be identical or different and are selected from among hydrogen, alkyl (C₁-C₆) o aryl; thiophenes such as, by way of non-limiting example, tetrahydrothiophene, methylthiophene, dimethylthiophene, benzothiophene or combinations thereof; mercaptans such as, by way of non-limiting example, methylmercaptan, ethylmercaptan, propylmercaptan, butylmercaptan or any combination thereof; carbonyl sulphide or any combination thereof. Preferably, the sulphurated compound may be hydrogen sulphide (H₂S), a dialkyl sulphide-type compound (R₂S, where R = methyl, ethyl, propyl or benzyl) or a combination thereof and, more preferably, is H₂S;
- the concentration of the sulphurated compound in the feed stream is comprised between 1 and 5,000 parts per million. Preferably, it is comprised between 20 and 200 parts per million;
- the reaction of the catalytic conversion of synthesis gas is carried out at a temperature comprised between 250ºC and 350ºC. Preferably, this temperature is comprised between 280ºC and 320ºC;
- the reaction of the catalytic conversion of synthesis gas is carried out at a total pressure comprised between 5.0 and 20.0 MPa. Preferably, total pressure is comprised between 7.5 and 15.0 MPa.

Throughout the description and claims, the word "comprises" and its variants do not aim to exclude other technical characteristics, additives, components or steps. For persons skilled in the art, other objects, advantages and characteristics of the invention will be partially inferred from the description and partially from the implementation of the invention. The following examples are provided by way of illustration and do not aim to limit the present invention.

### EXAMPLES

### Example I

In a particular embodiment of the present invention, the catalyst is obtained by firstly synthesizing a material containing Mo and Co according to the process disclosed in patents US6299760 and US6635599, and said material is subsequently modified by adding K. The preparation of said catalyst comprises the following steps: on one hand, 12.36 g of (NH₄)₆Mo₇O₂₄·4H₂O are dissolved in 250 ml of deionised water, the pH of said solution is adjusted to 9.8 by adding a 25% by weight aqueous ammonia solution and the resulting solution is heated to 90ºC in an oil bath under agitation; on the other hand, 20.37 g of Co(NO₃)₂·6H₂O are dissolved in 20.4 ml of deionised water. Next, the solution containing Co is added (0.5 ml/min.) to the solution containing Mo, which is maintained at 90ºC. After the addition, the resulting suspension is aged at 90ºC, under agitation, for 30 minutes. The resulting solid is isolated by hot filtration, washed with hot deionised water and dried at 100ºC for 12 hours. The solid thus obtained has an atomic ratio of Co/Mo = 1.2. Subsequently, the solid is impregnated with an aqueous solution containing the necessary amount of K₂CO₃ to obtain an atomic ratio of K/Mo = 0.5 and the resulting solid is dried at 80ºC for 10 hours.

### Example II

In another particular embodiment of the present invention, the catalyst is obtained by firstly synthesizing a material containing Mo and Co according to the process disclosed in patents US6299760 and US6635599, and said material is subsequently modified by adding K. The preparation of said catalyst comprises the following steps: on one hand, 6.18 g of (NH₄)₆Mo₇O₂₄·4H₂O are dissolved in 125 ml of deionised water, the pH of said solution is adjusted to 9.8 by adding a 40% by weight aqueous methylamine solution (CH₃NH₂) and the resulting solution is heated to 90ºC in an oil bath under agitation; on the other hand, 10.18 g of Co(NO₃)₂·6H₂O are dissolved in 10.25 ml of deionised water. Next, the solution containing Co is added (0.5 ml/min.) to the solution containing Mo, which is maintained at 90ºC. After the addition, the resulting suspension is aged at 90ºC, under agitation, for 30 minutes. The resulting solid is isolated by hot filtration, washed with hot deionised water and dried at 100ºC for 12 hours. The solid thus obtained has an atomic ratio of Co/Mo = 1.2. Subsequently, the solid is impregnated with an aqueous solution containing the necessary amount of K₂CO₃ to obtain an atomic ratio of K/Mo = 0.5 and the resulting solid is dried at 80ºC for 10 hours.

### Example III

In another particular embodiment of the present invention, the preparation of the catalyst comprises the following steps: on one hand, 6.18 g of (NH₄)₆Mo₇O₂₄·4H₂O are dissolved in 125 ml of deionised water, the pH of said solution is adjusted to 9.9 by adding a 40% by weight aqueous methylamine solution (CH₃NH₂) and the resulting solution is heated to 90ºC in an oil bath under agitation; on the other hand, 10.18 g of Co(NO₃)₂·6H₂O and 1.10 g of Zn(NO₃)₂·6H₂O are dissolved in 10.25 ml of deionised water. Next, the solution containing Co and Zn is added (0.5 ml/min.) to the solution containing Mo, which is maintained at 90ºC. After the addition, the resulting suspension is aged at 90ºC, under agitation, for 30 minutes. The resulting solid is isolated by hot filtration, washed with hot deionised water and dried at 100ºC for 12 hours. The solid thus obtained has an atomic ratio of Co/Mo = 1.1 and an atomic ratio of Zn/(Co+Mo) = 0.06. Subsequently, the solid is impregnated with an aqueous solution containing the necessary amount of K₂CO₃ to obtain an atomic ratio of K/Mo = 0.5 and the resulting solid is dried at 80ºC for 10 hours.

### Example IV

In another particular embodiment of the present invention, the preparation of the catalyst comprises the following steps: on one hand, 6.18 g of (NH₄)₆Mo₇O₂₄·4H₂O are dissolved in 125 ml of deionised water, the pH of said solution is adjusted to 9.8 by adding a 40% by weight aqueous methylamine solution (CH₃NH₂) and the resulting solution is heated to 90ºC in an oil bath under agitation; on the other hand, 10.18 g of Co(NO₃)₂·6H₂O and 0.95 g of Ga(NO₃)₃·H₂O are dissolved in 10.25 ml of deionised water. Next, the solution containing Co and Ga is added (0.5 ml/min.) to the solution containing Mo, which is maintained at 90ºC. After the addition, the resulting suspension is aged at 90ºC, under agitation, for 30 minutes. The resulting solid is isolated by hot filtration, washed with hot deionised water and dried at 100ºC for 12 hours. The solid thus obtained has an atomic ratio of Co/Mo = 1.2 and an atomic ratio of Ga/(Co+Mo) = 0.04. Subsequently, the solid is impregnated with an aqueous solution containing the necessary amount of K₂CO₃ to obtain an atomic ratio of K/Mo = 0.5 and the resulting solid is dried at 80ºC for 10 hours.

### Example V

By way of reference to the state of the art, a catalyst is prepared following the processes disclosed in patent US4831060, wherein a sulphided Mo precursor is used and a Mo sulphide promoted by Co is directly synthesized by precipitation in an aqueous medium with an acid pH and subsequent thermal activation. The catalyst preparation process comprises the following steps: on one hand, 3.65 g of (NH₄)₂MoS₄ are dissolved in 140 ml of deionised water; on the other hand, 2.10 g of Co(C₂H₃O₂)₂·4H₂O are dissolved in 60 ml of deionised water; on the other hand, 500 ml of a solution containing 30% by weight of acetic acid (CH₃COOH) is prepared. Next, the solution containing acetic acid is heated at 60ºC and the corresponding solutions containing Mo and Co are simultaneously added to said solution. The resulting solution is aged at 60ºC, under agitation, for 1 hour and the solid thus formed is isolated by hot filtration and dried at 30ºC for 10 hours. Next, the catalyst is calcined at 500ºC for 1 hour in a flow of N₂. The solid thus obtained has an atomic ratio of Co/Mo = 0.6. Next, the solid is impregnated with an aqueous solution containing the necessary amount of K₂CO₃ to obtain an atomic ratio of K/Mo = 0.8 and the resulting solid is dried at 80ºC for 10 hours.

### Example VI

In another embodiment of a material representative of the state of the art, the catalyst is obtained following the same process described in Example V, except that Cs is added instead of K, using the necessary amount of Cs₂CO₃ to obtain an atomic ratio of Cs/Mo = 0.8.

### Use of catalysts in a process for producing higher alcohols (C₂₊) through the catalytic conversion of synthesis gas

In a general comparison method, the catalysts prepared according to examples I to VI are applied to the catalytic conversion of synthesis gas. Catalytic tests are carried out in a high-capacity reactive system based on fixed-bed reactors (Avantium Technologies BV, Amsterdam). The process followed for the catalytic tests comprises a general methodology according to which 100-200 mg of the catalyst sieved to a particle size in the range of 50-150 µm are loaded into a cylindrical steel reactor having an inner diameter of 2-2.6 mm. The thermal activation treatment takes place in the reactor. All the catalysts (examples I to VI) are activated in a flow of N₂ at 300ºC for 1 hour. The catalysts prepared in accordance with the present invention (examples I to IV) are subjected to an additional sulphidation treatment at 400ºC for 3 hours in a flow of 10% (Vol.) H₂S/H₂. The catalysts prepared according to examples V and VI are not subjected to this sulphidation treatment as they are synthesized using sulphur-bearing precursors. On concluding the activation and sulphidation pretreatments (where applicable), the reactor is cooled to the reaction temperature (280-320ºC), pressurised in N₂ at 9.0 MPa and the circulating gas is substituted for a feed consisting of He:CO:H₂:H₂S (10%:45%:45%:50ppm), He being the reference inert gas. The reaction products are diluted in a N₂ stream and analysed by means of gas chromatography using two detectors (TCD and FID). Table 1 shows the catalytic results obtained from the catalysts prepared following examples I to VI under similar operating conditions.

**Table 1**

| Example | I | I | II | III | III | IV | V | V | VI |
|---|---|---|---|---|---|---|---|---|---|
| Temperature (ºC) | 300 | 310 | 310 | 300 | 310 | 310 | 300 | 320 | 300 |
| Pressure (MPa) | 9.0 | 9.0 | 9.0 | 9.0 | 9.0 | 9.0 | 9.0 | 9.0 | 9.0 |
| WHSV (h⁻¹)^{a} | 0.4 | 0.4 | 0.4 | 0.4 | 0.4 | 0.3 | 0.4 | 0.4 | 0.5 |
| Mole ratio H₂/CO | 1.0 | 1.0 | 1.0 | 1.0 | 1.0 | 1.0 | 1.0 | 1.0 | 1.0 |
| H₂S feed (ppm) | 50 | 50 | 50 | 50 | 50 | 50 | 50 | 50 | 50 |
| Conversion of CO (%) | 35.3 | 40.0 | 51.9 | 43.4 | 50.7 | 42.6 | 49.7 | 72.2 | 27.2 |
| Selectivity to CO₂ (%C) | 35.2 | 39.8 | 42.5 | 35.0 | 44.5 | 42.7 | 43.4 | 50.9 | 34.5 |

| Selectivities to products on CO₂-free basis (%C) | | | | | | | | | |
|---|---|---|---|---|---|---|---|---|---|
| CH₄ | 22.4 | 26.1 | 26.8 | 23.8 | 24.0 | 26.5 | 27.6 | 32.6 | 26.0 |
| Hydrocarbons C₂₊ | 4.9 | 6.0 | 7.7 | 4.6 | 7.0 | 7.7 | 22.6 | 38.3 | 3.7 |
| Methanol | 11.6 | 5.4 | 2.4 | 11.2 | 2.9 | 3.0 | 8.7 | 1.1 | 16.3 |
| Ethanol | 54.6 | 51.3 | 47.0 | 44.9 | 54.6 | 54.5 | 29.2 | 12.0 | 46.6 |
| Alcohols C₃₊ b | 6.5 | 11.5 | 3.1 | 15.4 | 11.7 | 8.4 | 11.7 | 15.9 | 7.2 |
| Productivity to ethanol (g/kg_{catalyst}·h)^{a} | 37.1 | 37.1 | 39.2 | 35.9 | 43.6 | 36.7 | 24.1 | 12.5 | 32.8 |

| | | | | | | | | | |
|---|---|---|---|---|---|---|---|---|---|
| ^{a} Based on the catalyst mass loaded into the catalytic reactor prior to the activation and sulphidation processes, where applicable. ^{b} "Alcohols C₃₊" mainly comprises n-propanol, iso-propanol, n-butanol, iso-butanol and n-pentanol. | | | | | | | | | |

The use of the catalysts prepared according to the present invention (examples I to IV) in the catalytic conversion of synthesis gas increases the selectivity of the reaction to higher alcohols (ethanol + alcohols C₃₊) to between 23% and 63%, as well as productivity to ethanol to between 32% and 81 %, with respect to catalysts prepared following state-of-the-art processes (examples V and VI) under the same operating conditions.

## Claims

1. A process for obtaining a sulphurated multi-metallic catalyst, **characterised in that** a solid comprising at least the following components is sulphurated:
C(i)C(ii)ₓC(iii)_{y}
being,
C(i) a component (i) selected from the list comprising molybdenum (Mo), tungsten (W) and any combination thereof,
C(ii) a component (ii) selected from the list of elements comprising at least one element belonging to groups 7 to 14 of the periodic table and any combination thereof,
C(iii) a component (iii) selected from the list of elements comprising groups 1 and 2 of the periodic table, lanthanides and any combination thereof,
"x" and "y" the mole ratios of C(ii) and C(iii) with respect to C(i), respectively, "x" being comprised between 0.1-10 and "y" between 0.2-10;
**characterised in that** the process for obtaining the solid that comprises C(i)C(ii)ₓC(iii)_{y} comprises at least the following stages:
a) Combining and reacting at least one compound of C(i) with at least one compound of C(ii) to obtain a solid;
b) Adding a compound of C(iii) to the solid of stage (a);
wherein stage (a) is carried out in an aqueous medium, the solid is obtained by precipitation and is separated before stage (b).

2. The process according to claim 1, wherein C(i) comprises at least Mo.

3. The process according to any of claims 1 or 2, wherein C(ii) comprises at least Co, Ni or any combination thereof.

4. The process according to claim 3, wherein C(ii) also comprises at least one element selected from the list that comprises Re, Ru, Rh, lr, Zn, Ga, In, Ge, Sn, La, Sm and any combination thereof.

5. The process according to any of claims 1 to 4, wherein C(iii) comprises Li, Na, K, Rb, Cs or any combination thereof.

6. The process according to claim 5, wherein C(iii) comprises K, Cs or any combination thereof.

7. The process according to any of the preceding claims, **characterised in that** the sulphuration stage is carried out by exposing the solid to a gas stream that comprises at least one sulphurated component.

8. The process according to claim 7, **characterised in that** the sulphurated compound is selected from the list that comprises: a compound with the formula R¹R²S, wherein R¹ and R² may be identical or different therebetween and are selected from among hydrogen, alkyl (C₁-C₆) or aryl; thiophenes; mercaptans, carbonyl sulphide; and any combination thereof.

9. The process according to any of claims 7 and 8, wherein the gas stream also comprises a gas selected from among H₂, N₂, noble gas, synthesis gas and any combination thereof.

10. The process according to any of claims 7 to 9, wherein the molar proportion of the sulphurated compound in the gas stream is comprised between 1 % and 85%.

11. The process according to claim 10, wherein the molar proportion of the sulphurated compound in the gas stream is comprised between 6% and 20%.

12. The process according to any of the preceding claims, wherein the sulphuration temperature is comprised between 200ºC and 750ºC.

13. The process according to claim 12, wherein the sulphuration temperature is comprised between 300ºC and 600ºC.

14. The process according to any of the preceding claims, **characterised in that** the mole ratio "x" is comprised between 0.2 and 2.

15. The process according to any of the preceding claims, wherein at least 60% of the molar mass of C(ii) is selected from among Co, Ni and any combination thereof.

16. The process according to any of the preceding claims, wherein the mole ratio "y" is comprised between 0.1 and 4.

17. The process according to claim 16, wherein the mole ratio "y" is comprised between 0.3 and 1.5.

18. The process according to any of the preceding claims, **characterised in that** the solid that comprises C(i)C(ii)ₓC(iii)_{y} also comprises at least one element selected from among carbon, nitrogen or combinations thereof.

19. The process according to claim 18, wherein the ratio between the moles of carbon and the moles of C(i) is less than 3.

20. The process according to any of the preceding claims, **characterised in that** the solid that comprises C(i)C(ii)ₓC(iii)_{y} is obtained by activation, preferably by means of thermal treatment, of a precursor that comprises: a compound of C(i); a compound of C(ii); and a compound of C(iii).

21. The process according to claim 20, **characterised in that** activation is carried out at a temperature comprised between 200°-C and 700°-C.

22. The process according to claim 21, **characterised in that** activation is carried out at a temperature comprised between 250ºC and 550ºC.

23. The process according to any of claims 21 and 22, wherein the activation stage is carried out under a sulphur-free gas stream.

24. The process according to claim 23, wherein the gas stream comprises air, N₂, noble gas, H₂, synthesis gas or any combination thereof.

25. The process according to any one of the preceding claims, wherein precipitation is carried out by adjusting the temperature, pH, solvent volume or any combination thereof.

26. The process according to claim 25, wherein precipitation is carried out by adjusting the temperature and pH.

27. The process according to any of claims 25 and 26, wherein the temperature and pH of an aqueous solution of the compound of C(i) are adjusted in stage (a) before combining with the compound of C(ii).

28. The process according to claim 27, wherein the pH is adjusted to between 8 and 13.

29. The process according to any of claims 25 to 28, wherein the pH is adjusted by adding at least one compound selected from the list that comprises ammonia, ammonium hydroxide, organic amines, compounds which are thermally decomposed releasing ammonia and any combination thereof.

30. The process according to any of the preceding claims, wherein the temperature is adjusted to between 50ºC and 120ºC in stage (a).

31. The process according to any of the preceding claims, wherein the compound of C(i) is ammonium heptamolybdate, ammonium metatungstate or any combination thereof.

32. The process according to any of the preceding claims, **characterised in that** the compound of C(ii) is a nitrate, chloride, carbonate, acetate or combinations thereof.

33. The process according to any of the preceding claims, **characterised in that** the compound of C(iii) is a carbonate, hydroxycarbonate, acetate, acetylacetonate, citrate, nitrate, chloride or any combination thereof.

34. The process according to claim 33, **characterised in that** the compound of C(iii) is a carbonate.

35. The process according to any one of the preceding claims, wherein a support is additionally suspended in the liquid medium in stage (a).

36. The process according to claim 35, wherein the suspended support is a metal carbide, oxide, carbon or any combination thereof.

37. The process according to claim 36, wherein the oxide is selected from the list that comprises a clay, SiO₂, TiO₂, Al₂O₃, ZrO₂, lanthanide element oxide and any combination thereof.

38. The process according to any one of the preceding claims, wherein the addition of the compound of C(iii) in stage (b) is carried out by impregnation of the solid using an aqueous solution of one or several precursor salts of C(iii).

39. The process according to any one of the preceding claims, wherein the addition of the compound of C(iii) in stage (b) is carried out by means of physical mixture with a solid compound of C(iii).

40. A catalyst obtainable by the process defined according to any one the preceding claims.

41. Use of the catalyst according to claim 40, in a process for producing higher alcohols (C₂₊) through the catalytic conversion of synthesis gas.

42. Use of the catalyst according to claim 41, wherein the higher alcohol (C₂₊) is ethanol.
